# EUROPEAN PATENT APPLICATION

(11) **EP 4 411 365 A1**
(43) Date of publication of application: **07.08.2024**
(21) Application number: 21958612.0
(22) Date of filing: 01.10.2021
(51) Int. Cl.: G01N 27/82, G01N 27/85, F17D 5/00

(54) **INTERNAL DUCT INTEGRITY INSPECTION EQUIPMENT USING MAGNETIC METAL MEMORY**

(71) Applicant: Pipeway Engenharia Ltda, 20940-080 Rio de Janeiro (BR)
(72) Inventor: SILVA, José Augusto Pereira da, 22620-300 Rio de Janeiro (BR); WEID, Jean Pierre von der, 22460-210 Rio de Janeiro (BR); LIMA, Vinicius de Carvalho, 22793-084 Rio de Janeiro (BR)
(74) Representative: J A Kemp LLP
(86) International application number: PCT/BR2021/050426
(87) International publication number: WO 2023/049975

(57) **Abstract**

An equipment is described to be moved through the inside of ducts (pig), carrying on-board instruments which basically comprise an electronics module (3), a battery module (4), MMM magnetic sensors (6) and an odometrical system (7, 8), and which are intended for detecting and measuring structural anomalies and stress concentration zones in rigid or flexible ducts made of a metallic material and located on land or in the depths of the sea (offshore) by non-destructive metal magnetic memory (MMM) testing.

## Description

### FIELD OF THE INVENTION

The present invention refers to equipment that travels through the interior of pipes (Pig) carrying instrumentation intended to detect, locate, and measure anomalies by non-destructive testing known as Metal Magnetic Memory (MMM).

### BACKGROUND

All industries from the lighter industry to heavier industry, which need to carry out non-destructive testing to inspect its premises use ducts and pipes, on land and/or under the sea ("offshore").

These ducts and pipes may be present in small diameters, multiple diameters, pipes, or ducts having thick walls, flexible metallic ducts, without taking into account the scenario which may be in a dry location or more than 2,000 meters under the sea, the environment naturally inaccessible to man without a subsea robot equipment.

From here any references to piping, pipes and tubular means, intended to transport a liquid or a gas from one point to another, provide they are of metal structure will be referred to generically by the term ducts, for simplification.

Whatever the scenario it is of paramount importance that the integrity of these ducts is inspected to allow the assurance of its operation without drawbacks or accidents.

In order to be possible to assess the conditions of the duct in general, equipment that originally was used to promote cleaning of deposited residues along its inner surface caused by the fluid transported is usually employed. Such equipment is known in the art by the English term "Pig", and this way it will be referred to hereinafter.

With technical evolution, the pigs had embodied into their bodies onboard electronics that feed, interpret and store much information collected by means of sensors of various types during their passage along the ducts, with respect to the integrity thereof.

The so-called instrumented pigs travel within inside the ducts driven by the pumped fluid itself and performing some type of measurement, e.g. a corrosion index on the wall of the duct. As to the detection method it can be by ultrasound, geometry, logging, temperature, laser ray, inertial and magnetic, such as MFL ("Magnetic Flux Leakage").

There are several examples in the art on the use of instrumented pigs, employing magnetic means to perform inspection:
- US 4,649,343-A discloses an electromagnetic inspection system to detect, *in situ,* the location and type of fault in a pipe of a small orifice relatively long, and thick-walled in a heat exchanger wherein a resulting magnetic field is induced in the pipe wall having selected direction and magnitude by vectorial addition of the magnetic fields produced by two magnetic field generators, at least one of which is carried by a scanner to cross the pipe and have an array of sensors mounted in close proximity to the pipe wall, generating a unique signal when the scanner passes in a fault in the pipe.
- US 5,454,276-A discloses a piping inspection pig that includes a triggering mechanism, a first field generator, and a first sensor section connected to the drive mechanism and are thereby carried helically over the piping in a clockwise rotation direction. A second field generator and a second sensor section are also connected to the drive mechanism and are thereby carried helically across the piping in a counter-clockwise direction. A data recorder is operatively connected to the first section of the sensor and to the second section of the sensor to record field break data from which a graph of anomalies in the piping may be generated as an intersection grid of signal paths provided by the first and second helical sensors.
- US 2011/095752-A1 relates to a piping inspection vehicle comprising a main body, which is carried through a piping by fluid flow against resilient cups. Resilient radial extensions carry a circumferential matrix of magnetic proximity sensors to detect the spacing between each sensor and the metal of the piping wall. The inclined wheels for engagement with the piping wall provide a way for distance measurement. The vehicle has onboard data storage through a memory housed in a pressure vessel.
- WO 96/13699 A2 discloses an instrumented inspection pig. The pig comprises a central mandrel; two support members attached to the central mandrel; two pairs of diametrically opposed radial sensors outputting a signal proportional to the distance between the inner wall of the pipe and the centerline of the pig through the radial sensor; an orientation sensor measuring rotation orientation of the pig relative to the gravity vector and generating data indicating the same; a processor receiving the signals from the radial sensors and the orientation sensor and storing the data therein indicating the distance between the inner wall and the axial centerline of the pig; and a power supply providing power to the rotational sensors and the processor. The bulk diameters measured by the respective pairs of radial sensors at any point for which data is available are calculated from distances measured by the radial sensors. Each bulk diameter is corrected for slope and then corrected orthogonally using the distance measurements from the radial sensors.
- WO 98/43062 A1 discloses an inspection tool or pig for traveling through a piping comprising a first conveyor connected by a universal joint to a second conveyor that can support a unit of inertial measurement, a data recording system, and odometer wheels. The first conveyor may be a magnetic flux leakage tool carrier including a rubber cup and a conical tip. A magnetic circuit for piping inspection may be constructed by separating magnets or poles along a certain length and coupling them to the piping wall by means of flexible steel brushes and completed by a support bar. Magnetic sensor arrays are repeated radially around the conveyor to provide complete circumferential coverage.
- Document WO2005/095943 A1 discloses a method and system for conducting in-line measurements of tensions on the walls of a pipeline by the continuous method of Barkhausen, it comprises an inspection pig including permanent magnets or electromagnets by direct current to generate a magnetic field that moves with the inspection pig through a pipeline, inductive sensors or other types of magnetic field sensors for reading Barkhausen noise signals generated by the moving magnetic field and associated instrumentation for amplifying, filtering, detecting and storing Barkhausen noise signals. The size of the sensors can be selected to correspond to the size of the defects being investigated. By comparing the trend data over time to determine changes in Barkhausen noise levels, a higher detection sensitivity can be achieved. The method may be particularly advantageous for use on inspection pigs that also use magnetic flux leakage to determine piping defects, since the magnetic flux leakage method also uses permanent magnets or electromagnets by direct current.
- WO 2005/106451 A1 discloses an instrumented pig and method of operating the same to determine the characteristics of a ferromagnetic piping through which it passes, including a pig body, first and second circumferential magnets, coaxial, spaced-apart of opposite polarities supported in the pig body and providing substantially complete magnetic saturation of an area of the piping between the magnets, first instruments arranged between the magnets to generate signals responsive to flow leakage, providing the first information as to anomalies on the interior and/or exterior surfaces of the piping, second instruments supported by the body of the pig arranged between the magnets and for generating signals that are responsive to eddy currents induced in the piping internal surface, providing second information as to anomalies on the interior surface of the pipeline, signal processing circuitry combining first and second signals and wherein the second instruments are energized only in response to the signals generated by the signal processing circuit.
- WO 2008/002202 A1 relates to the inspection of magnetic failures of steel ducts by studying magnetic leakage fields with the aid of probing devices moveable within a pipe. The inventive magnetic system comprises a row of elements of the magnetic system that are placed concentrically along the circumference of the body to be detected. Wherein each element comprises a pole shoe, a magnetic field source and a magnetic conductor that is made of at least one flexible magnetic tape coil and is secured by the side surfaces thereof between said shoes and the magnetic field source and the center axis of the coil is guided perpendicular to the direction of travel of the fault detector.
- WO 2014/163536 A1 relates to the measurement technology and may be used to diagnose the technical condition of metal conducts. The device comprises at least two magnetic field induction sensors of three components positioned at different height levels with respect to a piping, a first amplifier, a second amplifier, an analog-to-digital converter (ADC), a device to determine the difference between field induction values along the X, Y and Z axes, a controller, a memory unit, an information display device, a unit for determining the magnitude and direction of a full magnetic field induction vector measured by the first three-component sensor, a unit to determine the magnitude and direction of a full magnetic field induction vector measured by the second three-component sensor and a unit to determine the difference and the angle between the full field induction vectors measured by the first three-component sensor and the second three-component sensor. This invention allows to establish a full image of the magnetic field fluctuations, including the magnitude and shape thereof.
- CN1948961 A discloses a measurement of magnetic variables and method of detecting metal magnetic memory for ferromagnetic crack. It uses the magnetic voltage concentration indicator to detect the object, processes noise removal and Fourier transformation to the triple channel metal magnetic memory detection signal to gain its amplitude and phase value, confirming the position of the crack according to the phase sudden change, removing the amplitude value difference Delta between the maximum and the minimum, the largest delta Hp difference between the three amplitude values for the same sampling point on three channels, then the rate delta Hp / delta Lx of the two adjacent sampling points Hp, the difference between the absolute value and the distance, providing the quantization data of ferromagnetic cracks in accordance with the three values.
- EP3 842 796 A1 discloses a method of detecting and locating fault concentration and mechanical stress in ferromagnetic metal constructions. The method is based on employing two synchronized rotating disks containing at least 4 magnetic field strength sensors, and at least one sensor capable of detecting the earth's magnetic field, and an electromagnetic field generating source at a given frequency, chosen according to the dimensions of the duct inspected at the end of the duct. Field anomalies are detected and associated with the fault or structural stress.

Traditional methods and means of diagnosis (ultrasonic inspection, magnetic particle inspection, X-ray) as the above examples, are functional, widely employed in the industry, however, the detect faults already developed and fail to prevent sudden fatigue damage of equipment, the main reason of faults and emergency maintenance, or even a disaster.

As seen in WO2014/163536A1, EP3842796A1 and CN1948961A mentioned above, a method of diagnosis of equipment and structures based on metal magnetic memory usage has been developed and successfully implemented in practice, while having limited resolution yet. The MMM method joins the potential opportunities for non-destructive assays (END) and mechanic of fracture, because of which it has several significant advantages over other methods of inspecting industrial objects.

There are already devices that commercially analyze externally equipment and pipings that are apparent or even buried to a shallow depth, such as that operated by Polyinform company. However, such equipment depends on human operation and cannot access ducts at great depths.

For inspection of ducts, more particularly ducts in inaccessible places or aggressive to the human being, the ducts have features that make it more difficult to employ conventional external inspection techniques. There is a need for an apparatus that can perform duct inspection non-destructively and that provides a precise scenario of the anomalies present or potential, safely and reliably, and with high definition that is not performed by any of the techniques heretofore used.

The present invention provides anomalies detection equipment in metal ducts based on Metal Magnetic Memory, having the ability to provide high-resolution measurements on lines accessible or inaccessible to the human being. The invention eliminates the use of internal or external magnetic sources as well as rotating or mobile devices in the tool, thereby decreasing the complexity of the tool and its operating costs.

### SUMMARY OF THE INVENTION

It is an object of the invention the equipment travelling inside the ducts (Pig) carrying an instrumentation which is intended to detect and measure anomalies by means of non-destructive testing known as Metal Magnetic Memory (MMM) in high resolution. The equipment of the present invention presents three constructive variants according to the type of duct in which the non-destructive testing is performed.

A first objective is achieved by means of a conventional rigid Pig with a metal body and having at each end a cup in general of polyurethane fixed for coupling to the inner diameter of the duct and being propelled along this by fluid pressure. It is also fixed at the rear end to the second cup an odometer comprised of wheels in contact with the inner wall of the duct for recording position of the pig along the duct. A pressure capsule containing electronics and battery is rigidly attached to the body between the cups. The body also has a plurality of arms such as to cover the entire inner diameter of the duct. At the end of each arm MMM magnetic sensors are attached in abrasion resistant shoes by direct contact with the inner wall of the duct and read three axes to detect magnetic components present in the duct.

A second objective is achieved from a flexible Pig having a body of flexible material, such as polyurethane, and at each end, a cup generally of polyurethane secured for coupling and capable of conforming to a heterogeneous range of inner diameters of the duct and being propelled along thereof by fluid pressure. A pressure capsule containing electronics and battery is rigidly attached to the body between the cups. To the body also between the cups, a disc with a plurality of housings is attached to cover the entire inner diameter of the duct and, in each housing, MMM magnetic sensors encapsulated in abrasion-resistant shoes are attached by direct contact with the inner wall of the duct. These sensors perform three-axis reading to detect magnetic components present in the duct and may have data processed for contactless odometer positioning connecting to the electronics by means of cables. There is still a provision of a support for fixing a mechanical, magnetic, optical, acoustic, or other nature transducer to determine the odometer position of the tool.

A third objective is achieved with a pig having an integrally molded foamed body of dimensions to suit one or more diameters of a duct, it has a frustoconical front end structurally following a substantially cylindrical body, usually in polyurethane foam. At the rear end of the substantially cylindrical body and aligned with the longitudinal axis, a pressure capsule containing electronics, feed battery, and a positioning system without physical contact with the duct. Along the cylindrical surface of the body, a plurality of housings is molded into several helical distribution lines so as to cover the entire inner diameter of the duct and each housing is coupled to a magnetic sensor encapsulated in abrasion-resistant shoes by direct contact with the inner wall of the duct and take reading on three axes to detect magnetic components present in the duct.

The pressure capsule is designed to withstand the operating pressure of the piping. The electronics perform the functions of receiving, controlling, recording, and storing the data generated by the sensors in a memory of any nature, for example solid state. The battery has a charge with autonomy specific to an inspection operation. The capsule is connected to a multiplexed magnetic MMM sensor system via wiring encapsulated with pressure connectors and, in the case of the rigid pig there is the connection relative to the odometers.

The reading performed by each sensor is performed on up to three axes, being a longitudinal (X), a tangential (Y) and an orthogonal (Z). The number of sensors per each 2.54 cm (1 inch) of the inner circumference of the duct should be chosen such as to define the resolution of the measurement; at least 4 sensors characterize the detection of metallic faults, heterogeneity of structure and zones of stress concentration as high resolution, by distinguishing defects less than 1/4 of an inch. The higher the number of sensors per inch the higher the resolution.

### BRIEF DESCRIPTION OF THE FIGURES

FIG. 1 shows a side view a first embodiment for the equipment of the invention in a rigid pig.
FIG. 2 shows a side view a second embodiment for the apparatus of the invention in the form of a flexible pig.
FIG. 3 shows a side view a third embodiment for the apparatus of the invention in the form of a foam pig.
FIG. 4 shows a block diagram in which the connections between the signal inputs to the multiplexed integrated system and the connection thereof with the electronics module can be identified.

### DETAILED DESCRIPTION OF THE INVENTION

The features of the equipment for the internal inspection of duct integrity by means of Magnetic Memory of Metal (MMM), will be best appreciated from the detailed description which will follow, by way of example, associated with the drawings referenced below, which are an integral part of the present specification.

At the early 1980's, researchers in Russia observed stress induced magnetic fields in boiler tube defect areas in a power station, and developed some instruments for detecting the stress concentration zones in ferromagnetic components. Most metallic structures and equipment made of ferromagnetic materials is susceptible to "self-magnetization" by magnetic field of earth when under the influence of working loads.

The MMM method joins the potential opportunities for non-destructive testing (NDT) and fracture mechanics and, by this union presents several significant advantages over other industrial objects inspection methods.

The application of the MMM method does not require special magnetization devices, as observed in other tests, since the auto-magnetization phenomenon of equipment units and structures in the process of their operation is used. The MFL technique, for example, requires employing a magnetic head, which is avoided by applying the method now in reference, and which makes the tool more flexible and reduce costs.

The exact stress concentration points due to the working loads of the structure that are previously unknown are determined during their inspection.

This diagnostic method, based on the magnetic memory application of the metal, allows to perform an integral assessment of the state of a unit considering the quality of the metal, the actual operating conditions, and its structural characteristics. It comprises the inspection of the natural magnetic leakage field (*Self MFL, magnetic flux leakage*) of ferromagnetic materials, in a weak external magnetic field (usually the earth's magnetic field), at stress concentration zones and in metal anomalies. Natural magnetization and changes caused by anomalies and stress concentrations are part of the magnetic memory method.

The metallic structures and equipment of ferromagnetic materials are susceptible to "self-magnetization" as:
- SMFL (Self-MFL) by the earth's magnetic field when under the influence of working loads;
- When subjected to mechanical stresses there is a change in the magnetization in the material, Villary effect;
- Changes of magnetization occur when there is plastic deformation, known as Magneto-plasticity.

The diagnostic method, during evaluation, detects and sizes internal and external defects and magnetic concentration points. Current techniques such as Pig Palito, Pig DMR and Pig with "*Eddy-Currents*" sensors detect and size only internal defects. The MFL technique detects, without distinction, both internal and external defects.

The stress concentration points that are determined during an inspection, even if they do not have immediate failures, are mapped, and become a study and observation object for future failures by the operators of the equipment, and maintenance teams of ducts in general.

It is an object of the invention to provide an apparatus for displacement inside ducts (Pig) having instrumentation onboard, which is intended to detect and measure structural anomalies and stress concentration zones in metal ducts, whether rigid, flexible, on earth or deep sea ("*offshore*") by means of non-destructive testing of Metal Magnetic Memory (MMM) at high resolution, as well as an odometer positioning system.

The equipment of the present invention presents three constructive variants according to the type of duct in which the non-destructive testing is performed.

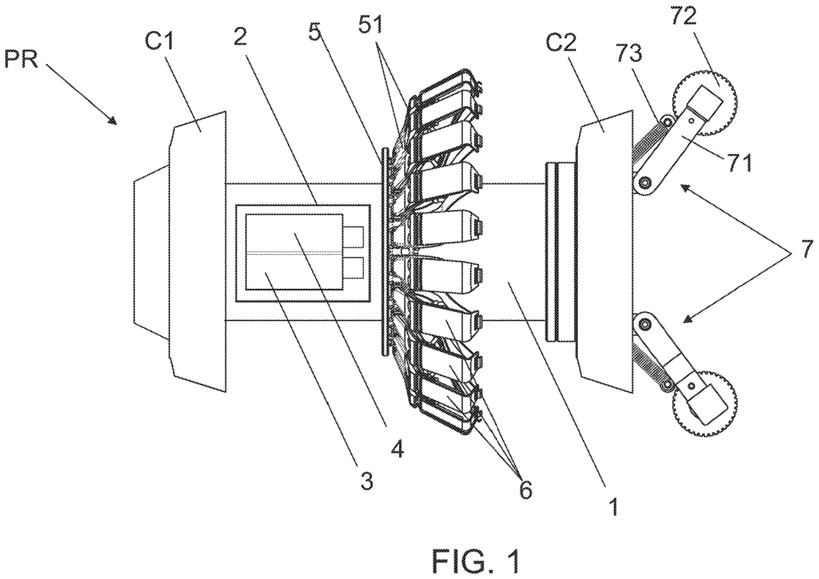

The first embodiment, which can be seen with the help of Figure 1, is a rigid pig (PR), more suitable for ducts having constant diameter and light curvatures, has a cylindrical body (1), rigid and metallic to which are connected in each ends thereof a first polyurethane cup (C1) and a second polyurethane cup (C2), sized for a given duct diameter (D) for centering and for promoting pressure differential generation necessary to move the pig along the duct by means of the pumped fluid itself, and comprises:
- a pressure capsule (2), which can be selected from: machined into the body (1) and rigidly attached to the body (1);
- an electronics module (3), installed inside the pressure capsule (2), comprised of a solid-state memory, is intended to collect and store data and records;
- a battery module (4), installed inside the pressure capsule (2) and electrically connected to the electronics module (3) is intended to feed with electrical power all the system;
- a crown (5), firmly attached to the body (1) between the polyurethane cups (C1) and (C2) has a plurality of radially expandable arms (51) and having a size capable of touching the inner wall of the duct (D);
- a plurality of magnetic sensors (6) MMM, where the quantity of sensors (6) depends on the diameter of the duct (D), may comprise at least 4 sensors for about 2.54 cm (1 inch) of inner circumference of the inner diameter of the duct (D), each sensor (6) or group of sensors is attached at the end of each arm (51) of the crown (5), is encapsulated in an abrasion-resistant shoe caused by the inner wall of the duct (D) with which it makes contact, performs the three-axis X, Y, Z reading referring to the detection of the components of the Normal and Tangential SMFL field of the duct (D) and transmits the reading data to the electronics module (3) by encapsulated cabling (CE) having pressure connectors (CP) (not shown in the figure);
- a contact odometer (7) is formed by two diametrically opposed sets attached to the end of the body (1) behind the cup (C2) where each set comprises a rod (71) with a wheel (72) at the outer end, having elastic tensioning, by spring (73) or similar device to maintain its contact with the internal walls of the duct (D); it is electronically connected to the electronics module (3) and electrically to the battery module (4), it serves to record the displacement of the rigid pig (PR) along the duct (D) and the position of an anomaly detected by the magnetic sensors MMM (6).

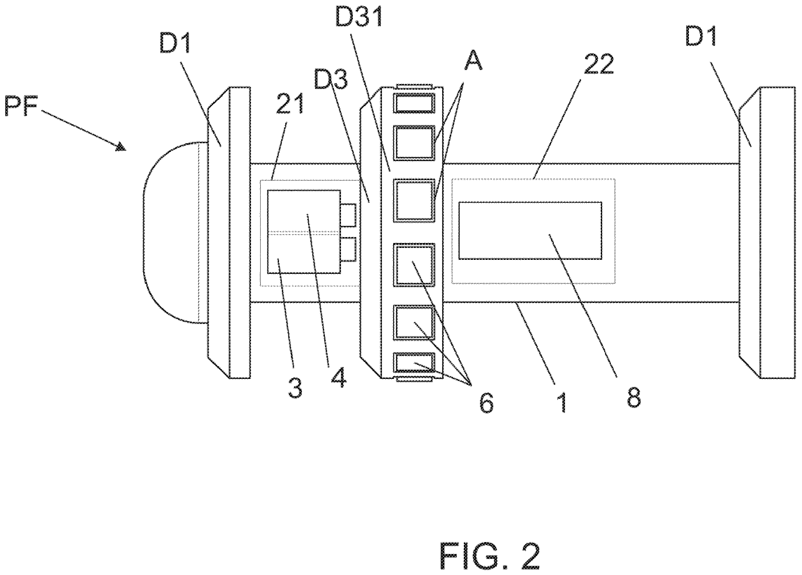

The second embodiment, which may be seen with the help of FIG. 2, is a flexible pig (PF), more appropriate for multi-diameter ducts, or those having sharp bends, formed by a single cylindrical polyurethane body (1) having at each end a first disk (D1) and a second disk (D2) being a structural part of the body (1), serve for centering and generating the pressure differential required to move the flexible pig (PF) by means of the pumped fluid itself, and comprises:
- a first pressure capsule (21) and a second pressure capsule (22) are excavated and formed in the single cylindrical body (1);
- an electronics module (3), installed within the first pressure capsule (21), comprised of a solid-state memory is intended to collect and store data and positional record;
- a battery module (4), installed inside the first pressure capsule (21) and electrically connected to the electronics module (3) is intended to feed with electric power all the system;
- an intermediate disc (D3) that is structural part of the body (1), is located between the first and second discs (D1 and D2), has structurally on its periphery a cylindrical outer extension (D31) capable of touching the inner wall of the duct (D) and at the surface of which there is a plurality of shaped housings (A);
- a plurality of magnetic sensors (6) MMM, where the quantity of sensors (6) depends on the diameter of the duct (D), may comprise at least 4 sensors for approximately 2.54 cm (1 inch) of the inner circumference of the inner diameter of the duct (D), each sensor (6) or group of sensors is attached to each of the shaped housings (A) at the outer cylindrical extension (D31) of the intermediate disc (D3), each sensor (6) or group of sensors is encapsulated in an abrasion-resistant shoe caused by the inner wall of the duct (D) with which it makes contact, performs the three-axis X, Y, Z reading referring to the detection of the components of the Normal and Tangential SMFL field of the duct (D) and transmits the reading data to the electronics module (3) by encapsulated cabling (CE) with pressure connectors (CP) (not shown);
- a contactless odometer system (8) is installed inside the second pressure capsule (22); it is electronically connected to the electronics module (3), electrically to the battery module (4) and serves to record the displacement of the flexible pig (PF) along the duct (D) and can be chosen to be an electromagnetic, topical, acoustic, and other transducer.

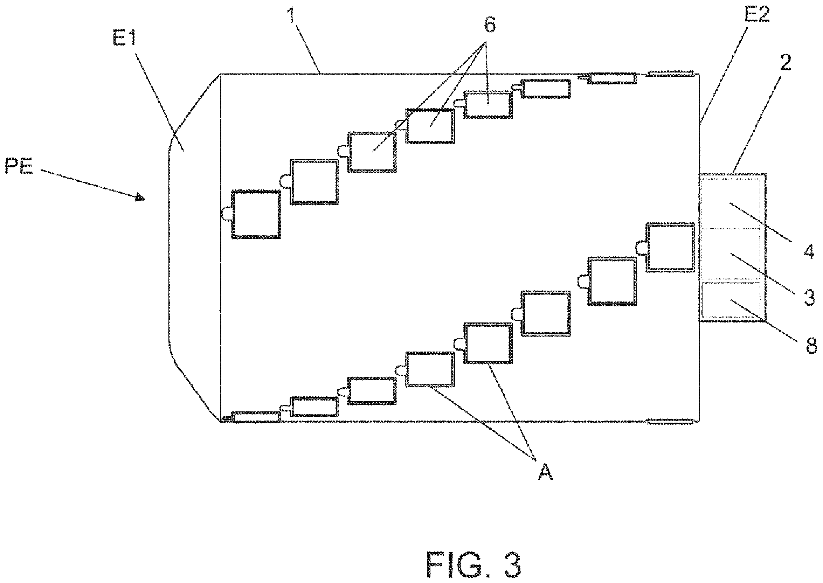

The third embodiment, which can be seen with the help of Figure 3, is a foam (PE) pig, suitable for any kind of duct, is formed by a single cylindrical polyurethane body (1), it presents a first end (E1) substantially in the shape of a frustum, the single body (1) having the diameter equal to the internal diameter of the duct and contacts with the latter throughout its length and presents a second straight-shaped end (E2) for generating the pressure differential required to move the foam pig (PE) by means of the pumped fluid itself, and comprises:
- a pressure capsule (2), rigidly attached to and aligned with the longitudinal axis of the single body (1) at the surface of the second end (E2);
- an electronics module (3), installed inside the pressure capsule (2), comprised of a solid-state memory is intended to collect and store data and positional record;
- a battery module (4), installed inside the pressure capsule (2) and electrically connected to the electronics module (3), is designed to feed electric power to all the system;
- a plurality of shaped housings (A) on the outer surface of the single body (1) are formed along a plurality of helical lines from the first end (E1) to the second end (E2) of the single body (1);
- a plurality of magnetic sensors (6) MMM, where the quantity of sensors (6) depends on the diameter of the conduct (D), may comprise at least 4 sensors for about 2.54 cm (1 inch) of inner circumference of the inner diameter of the duct (D), each sensor (6) or set of sensors is attached to each of the shaped housings (A), is encapsulated in an abrasion-resistant shoe caused by the inner wall of the duct (D) with which it contacts, performs the three-axis X, Y, Z readings referring to the detection of the components of the Normal and Tangential SMFL field of the duct (D) and transmits the reading data to the electronics module (3) by encapsulated cabling (CE) with pressure connectors (CP) (not shown);
- a contactless odometer system (8) is installed inside the pressure capsule (2); it is electronically connected to the electronics module (3) and electrically to the battery module (4), it serves to record the displacement of the foam pig (PE) along the duct (D) and may be chosen from an electromagnetic, optical, acoustic, and other transducer.

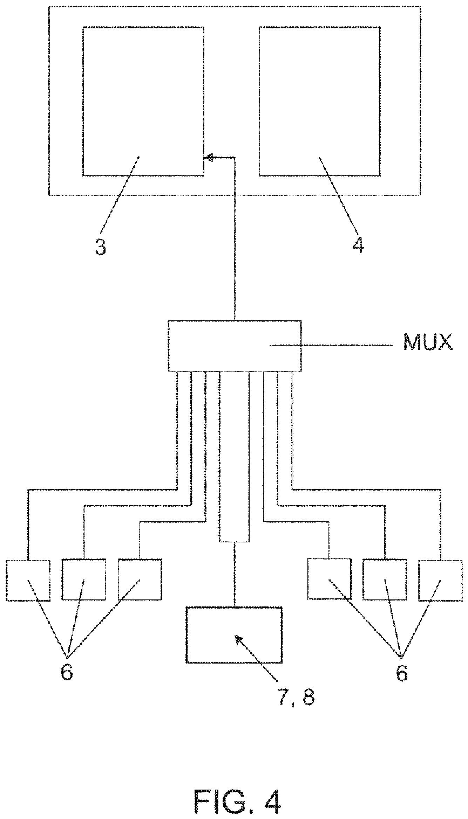

The electronics module (3), receives the data collected by the plurality of sensors (6) and by the systems: contact odometer (7) and contactless odometer system (8), by means of a multiplexed integrated system (MUX) and the scheme can be seen with the help of FIG. 4.

The density of magnetic sensors (6) MMM in the amount of at least four per 2.54 cm (1 inch) of inner circumference of the duct (D) each making a three-axis reading, it provides scanning of anomalies detection present and potential as magnetization points by concentration of stresses of high definition and allows to detect and size much smaller anomalies.

In flexible ducts (D), inspection is performed on the metallic wires constituting the outer layer of the tensile armor.

The description which has been made heretofore of the equipment for internal inspection of duct integrity by means of Magnetic Metal Memory (MMM), object of the present invention, is to be considered as possible embodiments only and, any particular characteristics introduced therein, such as increased resolution by increasing the density of sensors, employing different instrument transport systems, and sensors, or employing different combinations of sensors and odometers must be understood only as something that has been described to facilitate understanding. Accordingly, they may not in any way be considered as limiting the invention, which is only limited to the scope of the claims which follow.

## Claims

1. **- EQUIPMENT FOR THE INTERNAL INSPECTION OF DUCT INTEGRITY BY MEANS OF MAGNETIC MEMORY OF METAL** comprising equipment which moves inside ducts (Pig) **characterized by** instrumentation onboard, which is intended to detect, locate and measure structural anomalies and stress concentration zones in metal material ducts, whether they are rigid, flexible, on land, at deep sea by non-destructive testing of high resolution Metal Magnetic Memory (MMM), as well as an odometer positioning system.

2. **- EQUIPMENT FOR THE INTERNAL INSPECTION OF DUCT INTEGRITY BY MEANS OF MAGNETIC MEMORY OF METAL,** according to claim 1, in which a rigid pig (PR), more suitable for ducts having constant diameter and slight curvatures, it has a cylindrical, rigid and metallic body (1) to which are connected to each of its ends a first polyurethane cup (C1) and a second polyurethane cup (C2), sized for a given duct diameter (D) for centering and promoting the generation of pressure differential needed to move the pig along the duct by means of the pumped fluid itself, **characterized by** comprising:
- a pressure capsule (2), which can be selected from: machined into the body (1) and rigidly attached to the body (1);
- an electronics module (3), installed inside the pressure capsule (2), comprised of a solid-state memory, is intended to collect and store data and records;
- a battery module (4), installed inside the pressure capsule (2) and electrically connected to the electronics module (3) is intended to feed with electrical power all the system;
- a crown (5), firmly attached to the body (1) between the polyurethane cups (C1) and (C2) has a plurality of radially expandable arms (51) and having a size capable of touching the inner wall of the duct (D);
- a plurality of magnetic sensors (6) MMM, where the quantity of sensors (6) depends on the diameter of the duct (D), may comprise at least 4 sensors for about 2.54 cm (1 inch) of inner circumference of the inner diameter of the duct (D), each sensor (6) or group of sensors is attached at the end of each arm (51) of the crown (5), is encapsulated in an abrasion-resistant shoe caused by the inner wall of the duct (D) with which it makes contact, performs the three-axis X, Y, Z reading referring to the detection of the components of the Normal and Tangential SMFL field of the duct (D) and transmits the reading data to the electronics module (3) by encapsulated cabling (CE) having pressure connectors (CP);
- a contact odometer (7) is formed by two diametrically opposed sets attached to the end of the body (1) behind the cup (C2) where each set comprises a rod (71) with a wheel (72) at the outer end, having elastic tensioning, by spring (73) or similar device to maintain its contact with the internal walls of the duct (D); it is electronically connected to the electronics module (3) and electrically to the battery module (4), it serves to record the displacement of the rigid pig (PR) along the duct (D) and the position of an anomaly detected by the magnetic sensors MMM (6).

3. **- EQUIPMENT FOR THE INTERNAL INSPECTION OF DUCT INTEGRITY BY MEANS OF MAGNETIC MEMORY OF METAL,** according to claim 1, in which a flexible pig (PF), more appropriate for multi-diameter ducts, or more sharp bends, formed by a single cylindrical polyurethane body (1) having at each end a first disk (D1) and a second disk (D2) being a structural part of the body (1) serves for centering and generating the pressure differential necessary for moving the flexible pig (PF) by means of the pumped fluid itself, **characterized by** comprising:
- a first pressure capsule (21) and a second pressure capsule (22) are excavated and formed in the single cylindrical body (1);
- an electronics module (3), installed within the first pressure capsule (21), comprised of a solid-state memory is intended to collect and store data and positional record;
- a battery module (4), installed inside the first pressure capsule (21) and electrically connected to the electronics module (3) is intended to feed with electric power all the system;
- an intermediate disc (D3) that is structural part of the body (1), is located between the first and second discs (D1 and D2), has structurally on its periphery a cylindrical outer extension (D31) capable of touching the inner wall of the duct (D) and at the surface of which there is a plurality of shaped housings (A);
- a plurality of magnetic sensors (6) MMM, where the quantity of sensors (6) depends on the diameter of the duct (D), may comprise at least 4 sensors for approximately 2.54 cm (1 inch) of the inner circumference of the inner diameter of the duct (D), each sensor (6) or group of sensors is attached to each of the shaped housings (A) at the outer cylindrical extension (D31) of the intermediate disc (D3), each sensor (6) or group of sensors is encapsulated in an abrasion-resistant shoe caused by the inner wall of the duct (D) with which it makes contact, performs the three-axis X, Y, Z reading referring to the detection of the components of the Normal and Tangential SMFL field of the duct (D) and transmits the reading data to the electronics module (3) by encapsulated cabling (CE) with pressure connectors (CP);
- a contactless odometer system (8) is installed inside the second pressure capsule (22); it is electronically connected to the electronics module (3), electrically to the battery module (4) and serves to record the displacement of the flexible pig (PF) along the duct (D) and can be chosen to be an electromagnetic, topical, acoustic, and other transducer.

4. **- EQUIPMENT FOR THE INTERNAL INSPECTION OF DUCT INTEGRITY BY MEANS OF MAGNETIC MEMORY OF METAL,** according to claim 1, wherein a pig in which a foam pig (PE), suitable for any type of duct, is formed by a single cylindrical polyurethane body (1) having a first end (E1) in a substantially frustoconical shape, the single body (1) has the diameter equal to the inner diameter of the duct and contacts the latter over its entire length and presents a second end (E2) in a straight shape for generating the required pressure differential to moving the foam pig (PE) by means of the pumped fluid itself, **characterized by** comprising:
- a pressure capsule (2), rigidly attached to and aligned with the longitudinal axis of the single body (1) at the surface of the second end (E2);
- an electronics module (3), installed inside the pressure capsule (2), comprised of a solid-state memory is intended to collect and store data and positional record;
- a battery module (4), installed inside the pressure capsule (2) and electrically connected to the electronics module (3), is intended to feed electric power to all the system;
- a plurality of shaped housings (A) on the outer surface of the single body (1) are formed along a plurality of helical lines from the first end (E1) to the second end (E2) of the single body (1);
- a plurality of magnetic sensors (6) MMM, where the quantity of sensors (6) depends on the diameter of the conduct (D), may comprise at least 4 sensors for about 2.54 cm (1 inch) of inner circumference of the inner diameter of the duct (D), each sensor (6) or set of sensors is attached to each of the shaped housings (A), is encapsulated in an abrasion-resistant shoe caused by the inner wall of the duct (D) with which it contacts, performs the three-axis X, Y, Z readings referring to the detection of the components of the Normal and Tangential SMFL field of the duct (D) and transmits the reading data to the electronics module (3) by encapsulated cabling (CE) with pressure connectors (CP);
- a contactless odometer system (8) is installed inside the pressure capsule (2); it is electronically connected to the electronics module (3) and electrically to the battery module (4), it serves to record the displacement of the foam pig (PE) along the duct (D) and may be chosen from an electromagnetic, optical, acoustic, and other transducer.

5. **- EQUIPMENT FOR THE INTERNAL INSPECTION OF DUCT INTEGRITY BY MEANS OF MAGNETIC MEMORY OF METAL,** according to claims 1 and 2 and 3 and 4 **characterized in that** the electronics module (3), receiving the data collected by the plurality of magnetic sensors (6) MMM and by the systems: contact odometer (7) and the contactless electrical system (8), by means of a multiplexed integrated system (MUX).

6. **- EQUIPMENT FOR THE INTERNAL INSPECTION OF DUCT INTEGRITY BY MEANS OF MAGNETIC MEMORY OF METAL** according to claims 1 and 2 and 3 and 4 **characterized in that** the density of magnetic sensors (6) MMM in the amount of at least four per 2.54 cm (1 inch) of inner circumference of the inner diameter of the duct (D), each conducting a three-axis reading, allows the scanning of anomalies detection present and potential, as magnetization points, by concentration of stresses are of high definition and allows detecting and sizing anomalies with up to ¼ inch size.

7. **- EQUIPMENT FOR THE INTERNAL INSPECTION OF DUCT INTEGRITY BY MEANS OF MAGNETIC MEMORY OF METAL,** according to claims 1 and 2 and 3 and 4 **characterized in that** in flexible ducts (D) the inspection is performed on the metallic wires constituting the outer layer of the tensile armor.
